# EUROPEAN PATENT SPECIFICATION

(11) **EP 4 486 433 B1**
(45) Date of publication and mention of the grant of the patent: **05.11.2025**
(21) Application number: 23709475.0
(22) Date of filing: 22.02.2023
(51) Int. Cl.: A61N 1/05, A61B 17/34

(54) **LEAD EXTRACTION SYSTEM FOR RETAINED LEAD CONNECTORS**
LEITUNGSEXTRAKTIONSSYSTEM FÜR GEHALTENE LEITUNGSVERBINDER
SYSTÈME D'EXTRACTION DE SONDE POUR CONNECTEURS DE SONDE RETENUS

(30) Priority: 01.03.2022 US 202263268726 P
(43) Date of publication of application: 08.01.2025
(73) Proprietor: Medtronic, Inc., Minneapolis, Minnesota 55432 (US)
(72) Inventor: MARSHALL, Mark T., Minneapolis, Minnesota 55432 (US)
(74) Representative: Zimmermann & Partner Patentanwälte mbB
(86) International application number: PCT/IB2023/051629
(87) International publication number: WO 2023/166381

(56) References cited:
- WO-A2-2009/073754
- US-A1- 2011 022 057
- US-A1- 2021 338 984
- US-A1- 2022 047 292
- US-B2- 6 687 548
- US-B2- 9 339 269
- PETER BOREK P ET AL: "Pacemaker and ICD leads: Strategies for long-term management", JOURNAL OF INTERVENTIONAL CARDIAC ELECTROPHYSIOLOGY, KLUWER ACADEMIC PUBLISHERS, BO, vol. 23, no. 1, 8 April 2008 (2008-04-08), pages 59 - 72, XP019644041, ISSN: 1572-8595, DOI: 10.1007/S10840-008-9249-7

## Description

### BACKGROUND

Cardiac pacing systems commonly include an implantable medical device (IMD) such as an implantable pulse generator, commonly known as a pacemaker, electrically connected to the heart by at least one transvenous endocardial lead. An endocardial lead provides an electrical pathway between the pacemaker, connected to the proximal end of the lead, and endocardial tissue, in contact with the distal end of the lead. Endocardial tissue refers to a specific layer of tissue in the interior of the heart's chambers. Electrical pulses emitted by the pacemaker travel through the endocardial lead and stimulate the heart to deliver a prescribed therapy.

Endocardial leads are often placed in contact with the endocardial tissue by passage through a venous access, such as the subclavian vein, the cephalic vein, or one of its tributaries.

Transvenous leads may be introduced into a vein and maneuvered into contact with the heart, and placement of the leads does not require major thoracic surgery.

In some patients, it may become necessary to extract and replace an implanted lead. For example, a lead may need to be acutely replaced when unacceptable stimulation thresholds are measured during an implant procedure, when the lead fails, or when the endocardial tissue around the lead implantation site becomes infected.

In some lead extraction procedures, a locking stylet or compression coil may be attached to the lead, and a practitioner may apply traction to extract the lead from the endocardial tissue and remove the lead from the vasculature of the patient. In some examples, if the lead has been implanted for an extended period of time, scar tissue formed around the lead along the intravascular course and at the implantation site in the endocardial tissue can make the extraction procedure more difficult. In such cases, a tubular extraction sheath may be used to track over the length of the lead and monitored with fluoroscopy and/or transesophageal echocardiography may be used to dissect the fibrotic scar adhesions that prevent lead extraction. In some examples, a distal tip of the tubular extraction sheath may include a rotating cutting element, a laser, a plasma generator, or an electrocautery system to assist in dissecting the adhesions. Once the tubular extraction sheath has been advanced to the implantation site, the lead may be extracted through the sheath and removed from the body of the patient.

In some examples, an inside diameter of an extraction sheath is about 7 French (Fr) (2.3 mm) to as large as 13 Fr (4.3 mm), and smaller extraction sheaths are desirable to prevent injury to the vasculature of the patient. However, the proximal end of pacemaker leads typically have a relatively bulky connector assembly that plugs into, and makes an electrical connection with, the pacemaker. Standard connectors used to connect leads to pacemakers, such as IS-1, IS-4 and DF-4, have an outside diameter of about 14 Fr to 15 Fr (4.6 mm to 5.0 mm). When extracting a lead, the relatively large diameter of the connector can require that the lead be cut near the connector to allow insertion of the lead into the extraction sheath. Once cut, the lead requires additional preparation steps to bind the conductors to a suitable locking stylet or compression coil so that traction may be applied to the lead. Incorrect preparation of the severed conductors can decrease lead tensile strength and increases the chance of lead breakage. In addition, some lead designs can lose substantial lead strength when cut. In addition, cutting the lead to remove the bulky connector may require lead insulation and other protective layers overlying the lead conductor be immobilized by, for example, a ligation suture.

In some cases, practitioners have adopted a method that retains the bulky lead connectors. This method in turn requires an extraction sheath with a larger internal diameter to allow passage of the connector. While retaining the connector maintains the structural strength of the lead, the large disparity between the inner diameter of the extraction sheath and the outside diameter of the lead can cause higher complication rates for vascular damage. For example, in some cases the larger gap between the lead outside diameter and the lead inside diameter can pull the vascular wall into the sheath, which can increase the likelihood of a large vascular tear.
US 9,339,269 B2 relates to explanting electrode leads.

### SUMMARY

In general, the present disclosure is directed to a system and method for extracting a transvenous endocardial lead. The system and method of the present disclosure do not require cutting the lead or removal of the connector from the lead body. The extraction system of the present disclosure utilizes a lead extender with a collet configured to releasably attach to a pin on the lead connector. The extraction system of the present disclosure further includes a locking ring that moves over the collet and engages an outer surface of the collet to lock the collet onto the connector pin. To apply a force to the implanted lead such as, for example, traction or rotation, a practitioner can simply use a tool to manipulate the extension wire, and the lead conductor need not be cut or otherwise disturbed during the extraction procedure.

To accommodate the larger diameter of the lead with the retained connector, the collet on the connector pin of the connector, and the locking ring on the collet, the lead extraction system of the present disclosure further includes an extraction sheath with a sheath body having an internal diameter configured to allow insertion of the lead extractor, and a tapered distal tip. The tapered distal tip and a distal portion of the extraction sheath include a wall with an arrangement of contiguous openings configured to allow insertion of the extraction system.

Once the connector pin with the engaged collet and locking ring are inserted into the body of the extractor sheath, the extraction sheath may be advanced over the lead. Monitored with fluoroscopy and/or transesophageal electrocardiography, the extraction sheath may be maneuvered through the vasculature of the patient. The smaller diameter of the tapered distal tip reduces the difference in diameter between the inside diameter of the extraction sheath and the outside diameter of the lead, which can reduce the potential for vascular injury as the extraction sheath is manipulated and passed through the veins of the patient to the implant site where the lead is attached to the endocardial tissue. In various examples, the tapered tip of the extraction sheath may optionally have a dilating shape, or can include one or more tools to excise fibrotic scar adhesions around the lead such as rotational cutting tips, lasers, electrocautery, plasma, and the like.

In some examples, the endocardial lead extraction system of the present disclosure may optionally include a snare device with a wire loop that can be used to wrap about and securely retain at least one of the connector pin, the lead body, or the extension wire on the collet. In some examples, the snare device may be used by a practitioner to more efficiently apply traction or rotational force to extract the lead from the vasculature of the patient. In various examples, the snare device may be provided within the internal bore of the body of the extraction sheath, or may be external to the extraction sheath.

In some lead extraction procedures such as, for example, the extraction of temporary pacing leads, the snare device may be used with or without the lead extraction system described above, to assist a practitioner in applying traction or rotational force directly to the connector pin on the lead, or to the lead itself, to remove a lead from the vasculature of the patient.

In another aspect, the present disclosure is directed to methods for removing a transvenous endocardial lead using the lead extraction system, the snare device, or a combination thereof.

The lead extraction system of the present disclosure does not require cutting the lead, which can prevent lead damage or breakage as traction or rotational force is applied to the lead during extraction procedures. Maintaining lead integrity also means that the lead insulation and other supporting layers need not be cut or sutured during the extraction procedure. The collet in the extraction system of the present disclosure attaches directly to the connector on the lead, and the extension wire on the collet may be used for attachment of a locking stylet or compression wire, which avoids attaching these devices to the lead conductor itself. The distal tapered extraction sheath provides openings configured to allow insertion into the sheath body of the extension wire, the lead having a connector pin with the collet and locking ring attached thereto. The tapered distal end of the extraction sheath has a smaller diameter than the sheath body, and can be used to more easily navigate the vasculature of a patient using an imaging technique such as, for example, fluoroscopy and/or transesophageal echocardiography.

In one aspect, the present disclosure is directed to a system for extracting a transvenous endocardial lead, the transvenous endocardial lead including a lead body having a proximal end with a connector configured for electrical connection to an implantable medical device (IMD), and a distal end configured to contact endocardial tissue. The system includes a lead extender with a collet configured to releasably engage a pin of the connector, and a locking ring configured to maintain the engagement of the collet on the pin of the connector. The system further includes a lead extraction sheath having a sheath body with a tapered distal region, wherein a wall of the tapered distal region and a wall of the sheath body include an arrangement of openings configured to allow insertion of the lead extender and the body of the endocardial lead.

In another aspect, the present disclosure is directed to a method for extracting a transvenous endocardial lead from vasculature of a patient. The transvenous endocardial lead includes a lead body having a proximal end with a connector configured for electrical connection to an implantable medical device (IMD), and a distal end contacting endocardial tissue. In a lead extender having a collet and a locking ring, the method includes releasably attaching a second end of the collet to a pin of the connector, wherein a first end of the collet has an extension wire; sliding a locking ring over the collet and the extension wire to retain the collet on the pin of the connector, and inserting the extension wire, and the endocardial lead with the connector pin having the lead extender thereon into a lead extraction sheath having a sheath body with a tapered distal region. A wall of the tapered distal region and the wall of the sheath body include an arrangement of openings configured to allow insertion of the endocardial lead with the lead extender thereon.

In another aspect, the present disclosure is directed to a method for extracting a temporary transvenous endocardial lead from vasculature of a patient. The transvenous endocardial lead includes a lead body having a proximal end with a connector configured for electrical connection to an implantable medical device (IMD), and a distal end contacting endocardial tissue. The method includes attaching a snare element to the temporary endocardial lead; and exerting a force with the snare element to remove the temporary endocardial lead from the vasculature of the patient.

The details of one or more embodiments of the disclosure are set forth in the accompanying drawings and the description below. Other features, objects, and advantages of the techniques of the disclosure will be apparent from the description and drawings, and from the claims.

### BRIEF DESCRIPTION OF DRAWINGS

FIG. 1 is a conceptual drawing illustrating an example system that includes a temporary or permanent implantable medical device (IMD) coupled to implantable medical leads.
FIG. 2A is a perspective view of an example of a collet in a system for extracting a transvenous endocardial lead according to the present disclosure.
FIG. 2B is a cross-sectional view along line A-A of the collet of FIG. 2A.
FIG. 2C is a end view of the collet of FIG. 2A.
FIG. 2D is an enlarged cross-sectional view of the snap-fit connector on the collet of FIG. 2A.
FIG. 3A is an end view of a locking ring in the system for extracting a transvenous endocardial lead according to the present disclosure.
FIG. 3B is a cross-sectional view along line A-A of the locking ring of FIG. 3A.

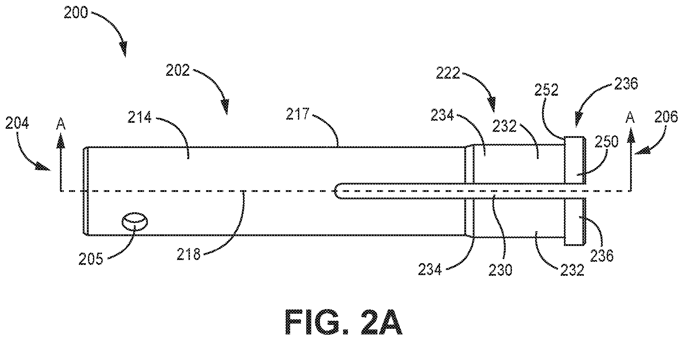

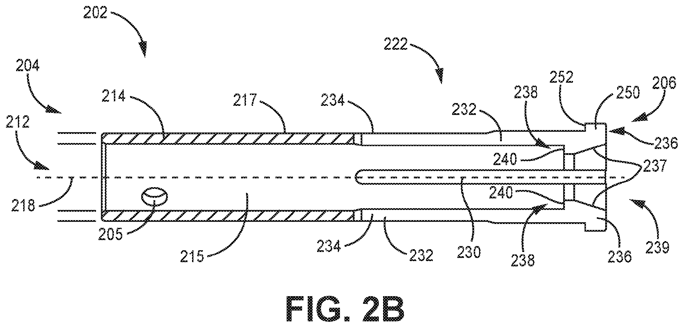

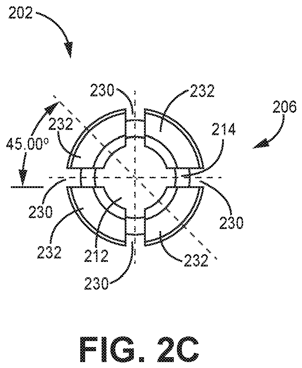

FIGS. 3C-1 to 3C-4 are photographs illustrating a method of attaching the collet of FIGS. 2A-D and the locking ring of FIGS. 3A-B to a connector on an endocardial lead.

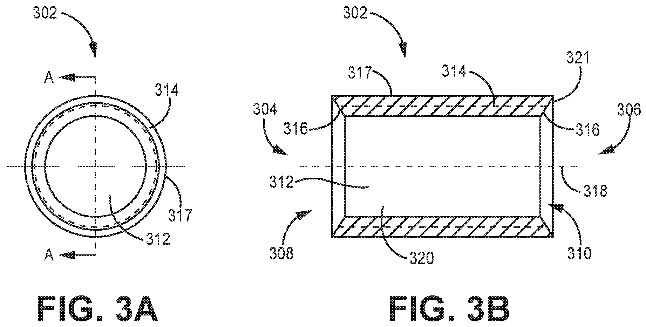

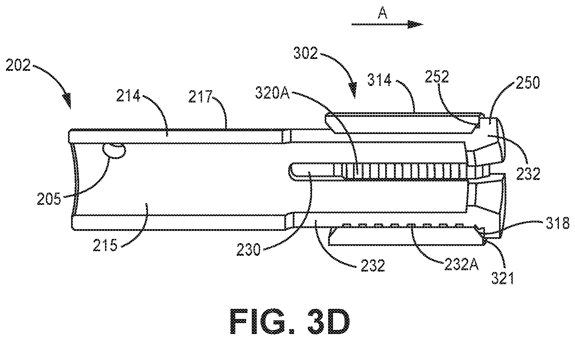

FIG. 3D is a cross-sectional side view of a collet and a threaded locking ring.

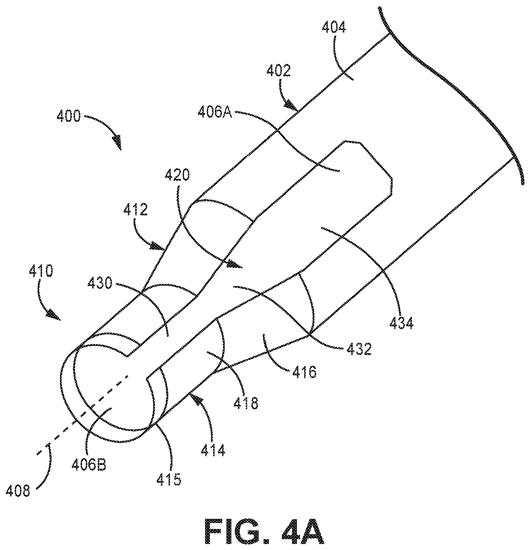

FIG. 4A is a schematic perspective view of a lead extraction sheath in a system for extracting a transvenous endocardial lead according to the present disclosure.
FIGS. 4B-1 to 4B-6 are photographs illustrating a method of inserting a transvenous endocardial lead with retained connector, collet and locking ring into the lead extraction sheath of FIG. 4A.

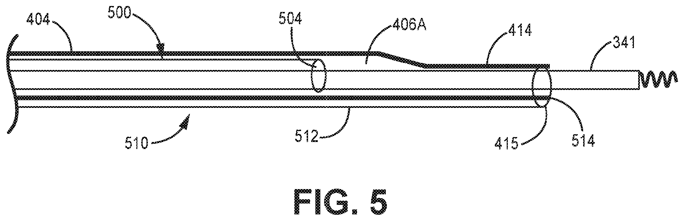

FIG. 5 is a schematic cut-away cross-sectional view of an example of the system including an optional snare device for extracting a transvenous endocardial lead according to the present disclosure.

Like symbols in the drawings indicate like elements.

### DETAILED DESCRIPTION

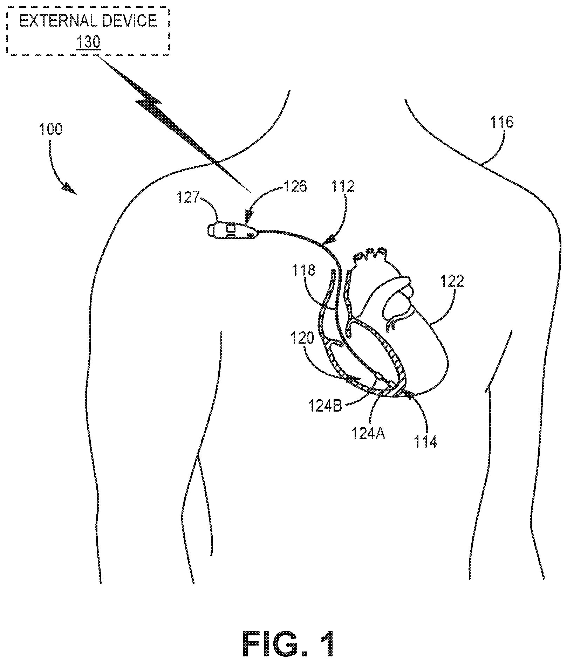

FIG. 1 is a conceptual diagram illustrating a portion of an example implantable medical device system 100. Implantable medical device system 100 may function as a single chamber, e.g., ventricular, pacemaker, as illustrated by the example of FIG. 1, or as dual-chamber pacemaker that delivers pacing to a heart 122 of patient 116. In some examples, the devices and methods of the present disclosure may be implemented in IMDs other than pacemakers, such as implantable monitors or neurostimulators.

In the example of FIG. 1, implantable medical device system 100 includes one or more implantable medical leads 112 electrically connected to the IMD 126. The implantable medical lead 112 includes an elongated lead body 118 with a distal portion 120 positioned at a target implantation site 114 within the heart 122 such as, for example, a ventricular wall. The lead 112 may be a unipolar, a bipolar, or a multipolar lead.

A clinician may maneuver the distal portion 120 of the lead 112 through the vasculature of patient 116 to position the distal portion 120 at or near the target site 114. For example, the clinician may guide distal portion 120 through the superior vena cava (SVC) to target site 114 on or in a ventricular wall of heart 122, e.g., at the apex of the right ventricle as illustrated in FIG. 1. Implantable medical device system 100 may include a delivery catheter and/or outer member (not shown), and implantable medical lead 112 may be guided and/or maneuvered within a lumen of the delivery catheter in order to approach target site 114.

The implantable medical lead 112 includes one or a plurality of electrodes. In the example of FIG. 1, the lead 112 includes electrodes 124A and 124B (collectively, "electrodes 124") configured to be positioned on, within, or near cardiac tissue at or near target site 114. In some examples, electrodes 124 provide pacing to heart 122. The electrodes 124 may be electrically connected to conductors (not shown in FIG. 1) extending through the lead body 118. In some examples, the conductors are electrically connected to therapy delivery circuitry of IMD 126, with the therapy delivery circuitry configured to provide electrical signals through the conductor to electrodes 124. Electrodes 124 may conduct the electrical signals to the target tissue of heart 122, causing the cardiac muscle, e.g., of the ventricles, to depolarize and, in turn, contract at a regular interval. Electrodes 124 may also be connected to sensing circuitry of IMD 126 via the conductors, and the sensing circuitry may sense activity of heart 122 via electrodes 124.

The electrodes 124 may have various shapes such as tines, helices, screws, rings, and so on. Again, although a bipolar configuration of lead 112 including two electrodes 124 is illustrated in FIG. 1, in other examples IMD 126 may be coupled to leads including different numbers of electrodes, such as one electrode, three electrodes, or four electrodes. In other examples (not shown in FIG. 1), the IMD 126 can be connected to two leads (atrium and right ventricle) or three leads (A, RV, LV), or other electrode or lead configurations.

The configuration of the therapy system 100 illustrated in FIG. 1 is merely one example. In other examples, a therapy system may include epicardial leads, subcutaneous, substernal, and/or patch electrodes instead of or in addition to the transvenous lead 112 illustrated in FIG. 1. Further, the IMD 126 need not be implanted within the patient 116. In examples in which the IMD 126 is not implanted in the patient 116, the IMD 126 may deliver therapies to the heart 122 via percutaneous leads that extend through the skin of patient 116 to a variety of positions within or outside of heart 122.

In one or more examples, IMD 126 includes electronic circuitry contained within an enclosure where the circuitry may be configured to deliver cardiac pacing. In the example of FIG. 1, the electronic circuitry within IMD 126 may include therapy delivery circuitry electrically coupled to electrodes 124. The electronic circuitry within IMD 126 may also include sensing circuitry configured to sense electrical activity of heart 122 via electrodes 124. The therapy delivery circuitry may be configured to administer cardiac pacing via electrodes 124, e.g., by delivering pacing pulses in response to expiration of a timer and/or in response to detection of the activity (or absence thereof) of the heart.

In some examples, the system 100 includes an optional programmer 130. For example, optional programmer 130 can be a handheld computing device such as a tablet or a phone, a computer workstation, or a networked computing device. The optional programmer 130 can include a user interface that receives input from a clinician, which can include a keypad and a suitable display such as, for example, a touch screen display, or a peripheral pointing device, such as a mouse, via which a user may interact with the user interface. The clinician may also interact with the programmer 130 remotely via a networked computing device.

After the implantable medical lead 112 and the electrodes 124 have been temporarily or more permanently implanted in the heart of a patient, the lead 112 and electrodes 124 may need to be removed due to structural defects, infections, or the need to upgrade a pre-existing system. After implantation for extended periods of time (for example, greater than about 1 year), chronic leads may develop a dense fibrotic and sometimes calcific process within the thin-walled venous structures or the endocardial surface of the heart or tricuspid valve, which can make the lead 112 and electrodes 124 difficult to extract. Complex lead extraction is associated with the risk of vascular injury by traction or perforation, causing tamponade, hemothorax, arteriovenous fistula, tricuspid valve disruption, or possibly pulmonary embolism, so simplifying or otherwise improving lead extraction techniques can have significant value for patient safety.

Extraction of leads can be performed by a variety of techniques, and in many cases simple traction or traction devices can be used to remove the lead from the vein of the patient. However, for chronic leads, various types of extraction sheaths, including mechanical, laser, electrosurgical, rotating threaded tip, and telescoping sheaths may be advanced over the lead and into the vein of the patient to remove the fibrotic scar tissue retaining the lead.

In various IMDs 126 (FIG. 1), standard male connector pins such as IS-1, IS-4, DF-4, SQ-1, and the like, have one or more structural features configured to engage a female receptacle in the IMD 126. These standard connector pins have a relatively large diameter compared to the body of the lead 112, and if left intact require an extraction sheath with a larger diameter to be used. However, as noted above, larger diameter extraction sheaths increase the likelihood of vascular injury, so smaller diameter extraction sheaths are preferred for most patients.

To utilize a smaller diameter extraction sheath, before advancing the extraction sheath over the lead, in some complex extraction cases the lead 112 to be extracted is cut to remove the connector pin. The cutting process can potentially damage and reduce the structural integrity of the lead, and in many procedures requires that the practitioner perform a number of additional steps to stabilize the severed lead before applying the extraction sheath.

The endocardial lead extraction system and method of the present disclosure include a lead extender that allows a practitioner to extract a transvenous lead from endocardial tissue of a patient without removing the connector pin of the implanted lead. A first component of the lead extender is a collet with a first end having an extension wire, and a second end including a snap-fit connector that releasably attaches to, or locks on, to a connector pin of the endocardial lead. After attachment of the collet to the connector pin, a second component of the lead extraction system, a locking ring, is moved over the collet to lock the collet into place on the connector pin. Once the collet is locked into place with the locking ring, a practitioner may apply a force, such as traction or rotation, to the lead via the extension wire, which avoids applying force directly to the body of the lead, and can reduce lead damage during extraction. The third component of the endocardial lead extraction system is a lead extraction sheath that includes a tapered distal tip with an arrangement of openings configured to allow the practitioner to advance the body of the sheath over the connector pin of the lead with the collet and locking ring attached thereto. The reduced diameter tapered distal tip may be more readily navigated through the veins of the patient to remove fibrotic adhesions, and the relatively small diameter of the tip relative to the body of the extraction sheath can reduce the likelihood of vascular damage.

Referring now to FIGS. 2A-2D, a first component of a lead extender 200 includes a collet 202 with a collet body 214 having a first open end 204, a second open end 206, and a generally cylindrical bore 212 extending along a longitudinal axis 218 between the first and second open ends 204, 206. The collet body 214 includes a wall 215 and an external surface 217, and the second end 206 includes an annular ring 250 forming an engagement surface 252.

The first end 204 of the collet 202 includes an aperture 205 extending through the wall 215 of the collet body 214. The aperture 205 provides an attachment site for an extension wire (not shown in FIGS. 2A-2D). Once the collet 202 is snap fit onto a connector pin and locked into place with a locking ring as explained in more detail below, a practitioner may apply a force, such as traction, rotation, or a combination thereof, to the extension wire. The extension wire provides a site for force application that does not require grasping or twisting the lead itself, and as such can prevent damage to the lead.

The second end 206 of the collet 202 includes a snap-fit connector region 222 formed by an arrangement of longitudinal slots 230 extending through the wall 215 of the collet body 214. In the example of FIGS. 2A-2D, the longitudinal slots 230 are elongate channels extending along about 50% of the length of the collet body 214, but any slot length may be used as needed to engage a particular shape or size of connector pin on an endocardial lead. In the example of FIGS. 2A-2D, the snap-fit connector region 222 includes 4 slots 230 arranged at 90° intervals about a circumference of the collet body 214, although any desired number and spacing of slots can be used. In addition, while the slots 230 in the example of FIGS. 2A-2D are arranged at regular angular intervals about the circumference of the collet body 214, the slots 230 may be arranged in any desired configuration to securely engage a selected connector pin shoulder, shape, or other structural feature.

The slots 230 divide the wall 215 of the collet body 214 into an arrangement of equally circumferentially spaced cantilevered arms 232, which reside between the slots 230. The cantilevered arms 232 each have a first end 234 extending from the body 214 of the collet 202, and a free second end 236. The cantilevered arms 232 are sufficiently flexible to expand outward in a direction generally normal to the longitudinal axis 218, which allows insertion of a connector pin of an endocardial lead into a flared opening 239. In the example of FIGS. 2A-2D, the second free end 236 of each arm 232 includes a wall 237 that opens to about, for example, an angle α = about 30°, to form the flared opening 239 to facilitate insertion of the connector pin on the endocardial lead.

The wall 237 on each cantilevered arm 232 includes an undercut 238 region forming a projecting tab 240. The tabs 240 extend generally normal to the longitudinal axis 218 of the collet 202, and may be configured to engage a distal shoulder of a connector pin on an endocardial lead. In various examples, the size and shape of the undercut 238 and tab 240 may be varied to fit a particular shoulder configuration or other structural feature of a connector pin, or to engage other features on the connector pin such as, for example, slots, apertures, and the like.

Once the connector pin of the endocardial lead is inserted into the flared opening 239, the collet 202 may be slidably moved distally along the connector pin, and the flexible cantilevered arms 232 move outward to allow insertion of the connector pin. The collet 202 is moved distally over the connector pin until the tabs 240 move over a shoulder on the connector pin. Once the shoulder of the connector pin moves past the tabs 240, the shoulder slides into the undercut regions 238, and the cantilevered arms 232 then snap into place to engage the connector pin. Once engaged, the tabs 240 and the undercut regions 238, as well as the clamping force exerted by the flexible cantilevered arms 232, prevent dislodgement of the connector pin from the collet 202.

The collet 202 may be made from a wide variety of materials including, but not limited to, polymeric materials, metals such as stainless steel, titanium, and the like. Suitable materials may be selected for the collet 202 to provide a desired amount of clamping force and static frictional engagement between the cantilevered arms 232 and a shoulder of a connector pin on the endocardial lead, as well as a desired amount of flexibility of the cantilevered arms 232.

Referring now to FIGS. 3A-3B, a second component of the lead extender 200 of the present disclosure includes a locking ring 302 with a locking ring body 314 having a first open end 304, a second open end 306, and a generally cylindrical bore 312 extending along a longitudinal axis 318 between the first and second open ends 304, 306. The first and second open ends 304, 306 each include respective flared openings 308, 310 with angled distal walls 316, 318.

The locking ring 302 may be made from a wide variety of materials including, but not limited to, polymeric materials, metals such as stainless steel, titanium, and the like. In some examples (not shown in FIGS. 3A-3B), an external wall 317 of the locking ring 302 may include a textured or knurled surface to allow a practitioner to easily grasp the locking ring 302 and slide the locking ring 302 over the collet 202. In some examples, the internal wall 320 of the locking ring 302 may be moved slidingly over the external surface 217 of the collet 202 until a distal end 321of the body 314 of the locking ring 302 abuts the engagement surface 252 on the annular ring 250 of the cantilevered arms 232 (FIGS. 2A-2D), thereby clamping the cantilevered arms 232 into position and securing the collet 202 on the connector pin on the endocardial lead.

Referring now to FIG. 3D, in some examples, an internal wall 320A of the locking ring 302 can be threaded to engage with a threaded surface 232A on the cantilevered arms 232 of the collet 202 (FIGS. 2A-2D). The internal wall 320A of the locking ring 302 may be moved slidingly over the external surface 217 of the collet 202, and the threads on the internal wall 320A may be engaged with the corresponding threads on the threaded surface 232A. The locking ring 302 may then be turned and advanced in a distal direction along the arrow A over the cantilevered arms 232 until the distal end 321 of the body 314 of the locking ring 302 abuts the engagement surface 252 on the annular ring 250 of the cantilevered arms 232, thereby clamping the cantilevered arms 232 of the collet 202 into position.

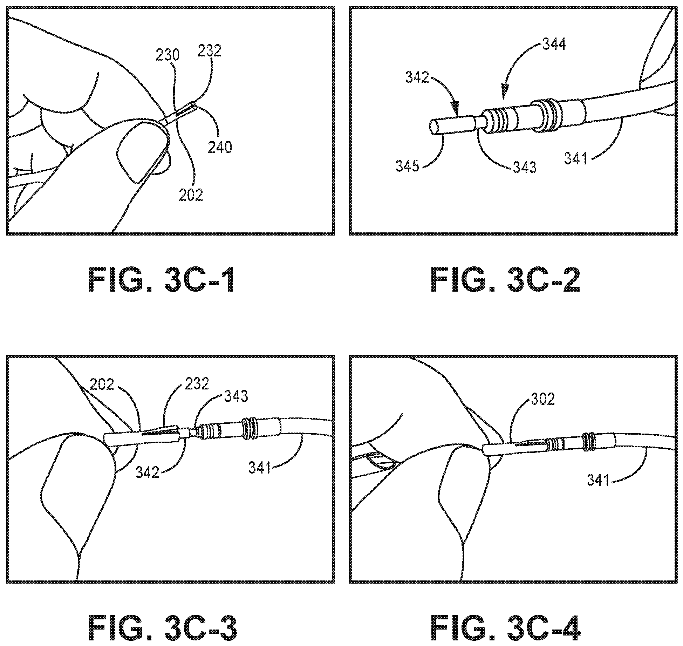

In operation, as shown in FIGS. 3C-1 and 3C-2, a collet 202 including longitudinal slots 230 and corresponding cantilevered arms 232 with projecting tabs 240 (FIGS. 2A-2D above), may be configured to slide over and engage a connector pin 342 on an IS-1 connector 344. The connector pin 342, which located on a proximal end of an endocardial lead body 341, includes a shoulder 343 on a distal end thereof, and an external surface 345. The collet 202 includes an extension wire (not shown in FIGS. 3C1-3C-4) engaged in the aperture 205 of the collet 202.

As shown in FIG. 3C-3, when the collet 202 is slidably advanced over and pressed onto the connector pin 342, the cantilevered arms 232 spread open and the connector pin 242 slides into the bore 212 of the collet 202. The tabs 240 on the arms 232 of the collet 202 then snap into place and engage the distal shoulder 343 of the connector pin 342, which holds the connector pin 342 securely within the bore 212 of the collet 202. The engagement of the tabs 240 of the collet 202 with the shoulder 343 of the connector pin 342, as well as clamping force exerted by the cantilevered arms 232, securely hold the connector pin 342 in position within the collet 202.

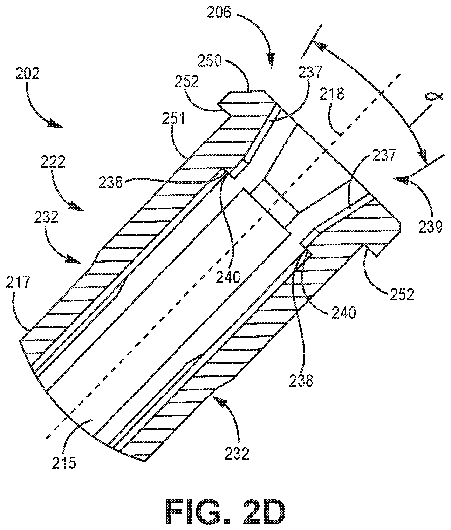

As shown in FIG. 3C-4, the locking ring 302 (FIGS. 3A-3B) may then slide in a distal direction over the external surface 217 of the collet 202 until the distal end 321 of the wall 314 thereof abuts the engagement surface 252 of the annular ring 250 on the collet 202. The locking ring 302 exerts a clamping force on an external surface 251 (FIG. 2D) of the cantilevered arms 232 of the collet 202 and prevents the arms 232 from spreading out and releasing the snap fit over the shoulder 343 of the connector pin 342. The extension wire engaged in the aperture 205 in the first end 204 of the collet 202 prevents the locking ring 302 from sliding in a proximal direction and disengaging from the external surface 217 of the collet 202.

Referring now to FIG. 4A, a second component of the system of the present disclosure, a lead extraction sheath 400, includes an elongate generally tubular sheath body 402 with a wall 404. The sheath body 402 includes an elongate bore 406A extending along a longitudinal axis 408 of the sheath 400. In some examples, the bore 406A of the sheath body 402 has an inside diameter of about at least about 13 Fr (4.3 mm) to allow passage of an endocardial lead with the proximal connector and lead extender (collet 202 and locking ring 302) attached. In some examples, the sheath body 402 has an outside diameter of about 16 Fr (5.3 mm). The sheath body 402 and bore 406A have a length sufficient to allow the sheath body to pass over the lead connector and lead extender and into the vasculature of a patient such that the extended lead fills the sheath body 402.

A distal end 410 of the extraction sheath body 402 includes a tapered region 412 that necks down into a distal tip 414. The distal tip 414 has a bore 406B that extends along the longitudinal axis 408 of the sheath body 402. In some examples, the bore 406B of the distal tip 414 has a inside diameter of about 6 Fr (2 mm) to about 12 Fr (4 mm). In one example, if the bore 406B has an inside diameter of 6 Fr (2 mm), the distal tip 414 has an outside diameter of about 8 Fr (2.6 mm). Since the distal tip 414 has an outside diameter that is approximately half the outside diameter of the sheath body 412, the distal tip 414 can be maneuvered more easily through the veins of a patient.

In some examples, the distal tip 414 can be made of an echogenic material to allow tracking of the tip 414 on an echocardiogram. In some embodiments, a distal end 415 of the distal tip 414 can optionally have a dilating shape or include tools to dislodge scar tissue and facilitate removal of the endocardial lead from the veins of the patient. Suitable tools can include, but are not limited to, fixed or rotating cutting heads, electrocautery elements, lasers, plasma generators, and the like.

Since the bore 406B has small inside diameter that will not allow passage of the large connector on a proximal end of the extended endocardial lead, the wall 404 of the sheath body 402 includes an arrangement of openings 420 to allow the extended endocardial lead to bypass the distal tip 414 and small bore 406B, and be inserted directly into the larger bore 406A. The arrangement of openings 420 includes contiguous openings extending through the wall 418 of the distal tip 414, the wall 416 of the tapered region 412, and the wall 404 of the sheath body 402. The arrangement of openings 420 may have any suitable shape, and in some examples includes contiguous slots arranged generally parallel to the longitudinal axis 408 of the sheath body 404.

In the embodiment of FIG. 4A, the arrangement of openings 420 includes a narrow slot 430 in the wall 418 of the distal tip 414. In some examples, the slot 430 has a diameter of at least about 1.5 mm. Moving in a direction away from the distal tip 414, the slot 430 extends into a wider slot 432 in the tapered region 412, and then extends into a substantially wider slot 434 in the wall of the sheath body 404. In some examples, the wider slot 434 has a width of at least about 4.1 mm, or more than twice the width of the slot 430 in the tapered region 414. In various examples, the widths of the slots 430, 432, and 434 may vary widely, and the slots may be the same or different.

In operation, the extractor sheath 400 of FIG. 4A may be used with the lead extender 200 and the components thereof, the collet 202 and the locking ring 302, to form a system for extracting a transvenous endocardial lead.

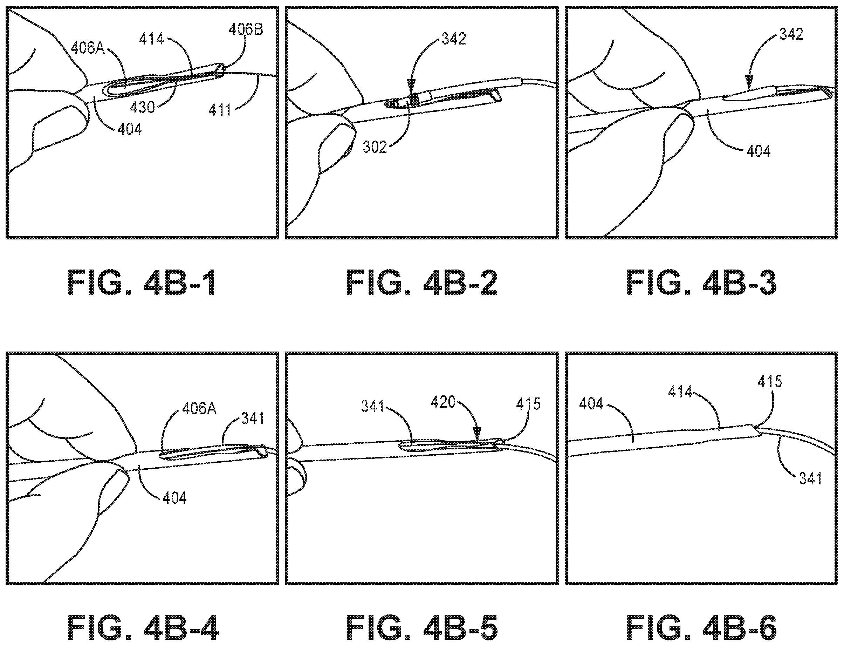

Referring now to FIG. 4B-1, a practitioner may grip the sheath body 404 of the extractor sheath 400, and insert the lead extension wire 411 attached to the collet 202 into the bore 408B and the slot 430 in the distal tip 414. The sheath body 404 may then be passed over the extension wire 411 until the extension wire 411 enters the bore 406A.

As shown in FIG. 4B-2, the extended endocardial lead including the collet 202 (not visible in FIG. 4B-2) snap-fit on the connector tip 342 and overlain by the locking ring 302, may then be inserted into the slot 434 in the sheath body 404. The large diameter of the internal bore 406A of the sheath body 404 allows easy passage of the extended lead body without binding or kinking.

As shown in FIG. 4B-3, the connector 342 may be fully inserted into the bore 406A of sheath body 404, and in FIG. 4B-4 the lead body 341 may also be inserted into the bore 406A.

As shown in FIGS. 4B-5 and 4B-6, the lead body 341 may then be easily inserted into the arrangement of slots 420, and extends from the distal end 415 of the distal tip 414. The distal end 415 of the distal tip 414 may then be inserted into the venous system of the patient and moved through the veins to remove fibrotic scar tissue surrounding the endocardial lead. The passage of the distal tip 414 may free the endocardial lead and facilitate removal of the lead from the veins of the patient.

In another example, the system for extracting a transvenous endocardial lead of the present disclosure may optionally include a snare device that may be used by a practitioner to securely grip any or all of the body of the endocardial lead, the connector tip, or the extension wire. As shown schematically in FIG. 5, a snare device 500 including an elongate thin pull wire 502 and a distal snare loop 504 may be used in the bore 406A of the sheath body 404 (FIG. 4A) to grip the body 341 of the endocardial lead. A practitioner may grip the pull wire 502 and cause the snare loop 504 to grip the lead body 341. Force, such as traction or rotation, may then be applied to the pull wire 502 to assist in drawing the lead body 341 into the bore 406A of the sheath body 404.

In another example schematically illustrated in FIG. 5, a snare device 510 may include an elongate thin pull wire 512 and a distal snare loop 514. The snare device 510 may be used outside the sheath body 404 such that the snare loop 514 grips the endocardial lead body 341 and draws the lead body into the distal tip 414 and the sheath body 404.

In another example, which is not shown in FIG. 5, the snare device 510 may be used alone, without the sheath body 404, to assist a practitioner in applying forces such as traction and rotation to a lead body to remove the lead body from the vasculature of the patient.

Various embodiments of the disclosure have been described. The present invention is set out in the appended claims.

## Claims

1. A system for extracting a transvenous endocardial lead, the transvenous endocardial lead (112) comprising a lead body (118) having a proximal end with a connector configured for electrical connection to an implantable medical device (IMD), and a distal end configured to contact endocardial tissue, the system comprising:
a lead extender (200), comprising:
a collet (202) configured to releasably engage a pin of the connector; and
a locking ring (302) configured to maintain the engagement of the collet on the pin of the connector; and
a lead extraction sheath (400) comprising a sheath body (402) with a tapered distal region (412), wherein a wall (416) of the tapered distal region and a wall (404) of the sheath body comprise an arrangement of openings (420) configured to allow insertion of the lead extender and the body of the endocardial lead.

2. The system of claim 1, wherein the collet has a first end (204) and a second end (206), and the second end of the collet is mechanically engaged with the pin of the connector.

3. The system of claim 2, wherein the second end of the collet comprises a snap-fit connector.

4. The system of any one of claims 2-3, wherein the second end of the collet comprises an arrangement of cantilevered arms and each cantilevered arm (232) in the arrangement of cantilevered arms is separated by a longitudinal slot.

5. The system of claim 4, wherein the collet comprises an arrangement of four cantilevered arms spaced at 90° about a circumference of the second end of the collet.

6. The system of claim of any one or more of claims 4-5, wherein a distal end of the at least some of the cantilevered arms comprises an undercut (238) configured to engage a shoulder of the pin of the connector, and optionally at least some of the undercuts form a tab (240), and wherein the tabs extend in a direction normal to a longitudinal axis of the collet.

7. The system of any one of claims 1-6, wherein the tapered distal region comprises a first slot that extends parallel to a longitudinal axis of the sheath body.

8. The system of any one of claims 1-7, wherein a distal region of the sheath body comprises a wall with a second slot contiguous with the first slot in the tapered distal region and, optionally, the second slot extends parallel to the longitudinal axis of the body of the extraction sheath.

9. The system of claim 8, wherein a width of the second slot is greater than a width of the first slot and, optionally, the width of the first slot is at least about 1.5 mm.

10. The system of any one of claims 1-9, wherein an inside diameter of the sheath body has a diameter of at least about 4.3 mm (about 13 Fr).

11. The system of any one of claims 1-10, wherein an inside diameter of the tapered region of the extraction sheath is at least about 2 mm (about 6 Fr).

12. The system of any one of claims 1-11, wherein a distal region of the tapered distal tip of the sheath body comprises at least one of a rotational cutting element, a laser, an electrocautery element, a plasma element, a dilating cutting tip, and combinations thereof.

13. The system of any one of claims 1-12, further comprising a snare element (500) configured to engage a proximal end of the endocardial lead and, optionally, the snare element is configured to engage the pin of the connector.

14. The system of any one of claims 1-13, wherein the first end of the collet comprises an aperture (205), and an extension wire (411) is attached to the collet within the aperture.

15. The system of any one of claims 1-14, further comprising a snare element (500) configured to engage the extension wire connected to the collet and, optionally, the snare element resides within the extraction sheath.

## Patentansprüche

1. System zum Herausziehen einer transvenösen endokardialen Leitung, wobei die transvenöse endokardiale Leitung (112) einen Leitungskörper (118) umfasst, der ein proximales Ende mit einem Verbinder, der zur elektrischen Verbindung mit einer implantierbaren medizinischen Vorrichtung (IMD) ausgestaltet ist, und ein distales Ende aufweist, das zum Kontaktieren von endokardialem Gewebe ausgestaltet ist, wobei das System Folgendes umfasst:
eine Leitungsverlängerung (200), die Folgendes umfasst:
eine Spannzange (202), die zur lösbaren Ineingriffnahme eines Stifts des Verbinders ausgestaltet ist, und
einen Sicherungsring (302), der dazu ausgestaltet ist, den Eingriff der Spannzange an dem Stift des Verbinders aufrechtzuerhalten, und
eine Leitungsherausziehhülse (400), die einen Hülsenkörper (402) mit einem sich verjüngenden distalen Bereich (412) umfasst, wobei eine Wand (416) des sich verjüngenden distalen Bereichs und eine Wand (404) des Hülsenkörpers eine Anordnung von Öffnungen (420) umfassen, die dazu ausgestaltet sind, ein Einführen der Leitungsverlängerung und des Körpers der endokardialen Leitung zu gestatten.

2. System nach Anspruch 1, wobei die Spannzange ein erstes Ende (204) und ein zweites Ende (206) aufweist und das zweite Ende der Spannzange mechanisch mit dem Stift des Verbinders in Eingriff steht.

3. System nach Anspruch 2, wobei das zweite Ende der Spannzange einen Schnappverbinder umfasst.

4. System nach einem der Ansprüche 2 - 3, wobei das zweite Ende der Spannzange eine Anordnung von auskragenden Armen umfasst und jeder auskragende Arm (232) in der Anordnung von auskragenden Armen durch einen Längsschlitz getrennt ist.

5. System nach Anspruch 4, wobei die Spannzange eine Anordnung von vier auskragenden Armen umfasst, die um 90° um einen Umfang des zweiten Endes der Spannzange beabstandet sind.

6. System nach einem oder mehreren der Ansprüche 4 - 5, wobei ein distales Ende der mindestens einigen der auskragenden Arme eine Hinterschneidung (238) umfasst, die zur Ineingriffnahme einer Schulter des Stifts des Verbinders ausgestaltet ist, und optional mindestens einige der Hinterschneidungen eine Lasche (240) bilden, und wobei sich die Laschen in einer orthogonal zu einer Längsachse der Spannzange verlaufenden Richtung erstrecken.

7. System nach einem der Ansprüche 1 - 6, wobei der sich verjüngende distale Bereich einen ersten Schlitz umfasst, der sich parallel zu einer Längsachse des Hülsenkörpers erstreckt.

8. System nach einem der Ansprüche 1 - 7, wobei ein distaler Bereich des Hülsenkörpers eine Wand mit einem zweiten Schlitz umfasst, der an den ersten Schlitz in dem sich verjüngenden distalen Bereich angrenzt, und sich optional der zweite Schlitz parallel zu der Längsachse des Körpers der Herausziehhülse erstreckt.

9. System nach Anspruch 8, wobei eine Breite des zweiten Schlitzes größer als eine Breite des ersten Schlitzes ist und optional die Breite des ersten Schlitzes mindestens etwa 1,5 mm beträgt.

10. System nach einem der Ansprüche 1 - 9, wobei ein Innendurchmesser des Hülsenkörpers einen Durchmesser von mindestens etwa 4,3 mm (etwa 13 Ch) aufweist.

11. System nach einem der Ansprüche 1 - 10, wobei ein Innendurchmesser des sich verjüngenden Bereichs der Herausziehhülse mindestens etwa 2 mm (etwa 6 Ch) beträgt.

12. System nach einem der Ansprüche 1 - 11, wobei ein distaler Bereich der sich verjüngenden distalen Spitze des Hülsenkörpers ein Rotationsschneidelement und/oder einen Laser und/oder ein Elektrokauterisationselement und/oder ein Plasmaelement und/oder eine Dilatationsschneidspitze und/oder Kombinationen davon umfasst.

13. System nach einem der Ansprüche 1 - 12, ferner umfassend ein Schlingenelement (500), das zur Ineingriffnahme eines proximalen Endes der endokardialen Leitung ausgestaltet ist, und optional ist das Schlingenelement zur Ineingriffnahme des Stifts des Verbinders ausgestaltet.

14. System nach einem der Ansprüche 1 - 13, wobei das erste Ende der Spannzange eine Öffnung (205) umfasst und ein Verlängerungsdraht (411) an der Spannzange innerhalb der Öffnung angebracht ist.

15. System nach einem der Ansprüche 1 - 14, ferner umfassend ein Schlingenelement (500), das zur Ineingriffnahme des mit der Spannzange verbundenen Verlängerungsdrahts ausgestaltet ist, und optional befindet sich das Schlingenelement innerhalb der Herausziehhülse.

## Revendications

1. Système d'extraction de sonde endocardique transveineuse, la sonde endocardique transveineuse (112) comprenant un corps de sonde (118) ayant une extrémité proximale pourvue d'un connecteur conçu pour une connexion électrique à un dispositif médical implantable (IMD), et une extrémité distale conçue pour entrer en contact avec un tissu endocardique, le système comprenant :
un dispositif d'extension de sonde (200) comprenant :
une pince (202) conçue pour venir en prise amovible avec une broche du connecteur ; et
une bague de verrouillage (302) conçue pour maintenir la mise en prise de la pince sur la broche du connecteur ; et
une gaine d'extraction de sonde (400) comprenant un corps de gaine (402) présentant une région distale effilée (412), une paroi (416) de la région distale effilée et une paroi (404) du corps de gaine comprenant un agencement d'ouvertures (420) conçues pour permettre l'insertion du dispositif d'extension de sonde et du corps de la sonde endocardique.

2. Système selon la revendication 1, la pince comprenant une première extrémité (204) et une seconde extrémité (206), et la seconde extrémité de la pince venant en prise mécaniquement avec la broche du connecteur.

3. Système selon la revendication 2, la seconde extrémité de la pince comprenant un connecteur à ajustement par encliquetage.

4. Système selon l'une quelconque des revendications 2 et 3, la seconde extrémité de la pince comprenant un agencement de bras en porte-à-faux et chaque bras en porte-à-faux (232) dans l'agencement de bras en porte-à-faux étant séparé par une fente longitudinale.

5. Système selon la revendication 4, la pince comprenant un agencement de quatre bras en porte-à-faux espacés à 90 ° autour d'une circonférence de la seconde extrémité de la pince.

6. Système selon l'une quelconque ou plusieurs des revendications 4 et 5, une extrémité distale d'au moins certains des bras en porte-à-faux comprenant une contre-dépouille (238) conçue pour venir en prise avec un épaulement de la broche du connecteur, et éventuellement au moins certaines des contre-dépouilles formant une patte (240), et les pattes s'étendant dans une direction normale à un axe longitudinal de la pince.

7. Système selon l'une quelconque des revendications 1 à 6, la région distale effilée comprenant une première fente qui s'étend parallèlement à un axe longitudinal du corps de gaine.

8. Système selon l'une quelconque des revendications 1 à 7, une région distale du corps de gaine comprenant une paroi pourvue d'une seconde fente contiguë à la première fente dans la région distale effilée et, éventuellement, la seconde fente s'étendant parallèlement à l'axe longitudinal du corps de la gaine d'extraction.

9. Système selon la revendication 8, la largeur de la seconde fente étant supérieure à la largeur de la première fente et, éventuellement, la largeur de la première fente étant d'au moins environ 1,5 mm.

10. Système selon l'une quelconque des revendications 1 à 9, un diamètre intérieur du corps de gaine ayant un diamètre d'au moins environ 4,3 mm (environ 13 Fr).

11. Système selon l'une quelconque des revendications 1 à 10, un diamètre intérieur de la région effilée de la gaine d'extraction étant d'au moins environ 2 mm (environ 6 Fr).

12. Système selon l'une quelconque des revendications 1 à 11, une région distale de la pointe distale effilée du corps de gaine comprenant au moins l'un d'un élément de coupe rotatif, d'un laser, d'un élément d'électrocautérisation, d'un élément plasma, d'une pointe de coupe dilatatrice et de combinaisons de ceux-ci.

13. Système selon l'une quelconque des revendications 1 à 12, comprenant en outre un élément anse (500) conçu pour venir en prise avec une extrémité proximale de la dérivation endocardique et, éventuellement, l'élément anse étant conçu pour venir en prise avec la broche du connecteur.

14. Système selon l'une quelconque des revendications 1 à 13, la première extrémité de la pince comprenant une ouverture (205), et un fil d'extension (411) étant fixé à la pince à l'intérieur de l'ouverture.

15. Système selon l'une quelconque des revendications 1 à 14, comprenant en outre un élément anse (500) conçu pour venir en prise avec le fil d'extension connecté à la pince et, éventuellement, l'élément anse se trouvant à l'intérieur de la gaine d'extraction.
